# EUROPEAN PATENT APPLICATION

(11) **EP 2 660 779 A2**
(43) Date of publication of application: **06.11.2013**
(21) Application number: 13166436.9
(22) Date of filing: 03.05.2013
(51) Int. Cl.: G06T 11/00

(54) **Apparatus and method for generating image in positron emission tomography**

(30) Priority: 03.05.2012 KR 20120047128; 16.10.2012 KR 20120115025
(71) Applicant: Samsung Electronics Co., Ltd, Gyeonggi-do 443-742 (KR)
(72) Inventor: Park, Byung-kwan, Gyeonggi-do (KR); Yi, Jae-mock, Gyeonggi-do (KR); Song, Tae-yong, Gyeonggi-do (KR)
(74) Representative: Grünecker, Kinkeldey, Stockmair & Schwanhäusser

(57) **Abstract**

A method and apparatus generate an image in positron emission tomography (PET). The method and apparatus are configured to divide detected signals into sections at time intervals. The detected signals are emitted from tracers introduced into a target. The method and apparatus are also configured to generate unit signals for each of the sections by accumulating the divided signals at each respective section. The method and apparatus are further configured to classify the unit signals into groups based on characteristics of each of the unit signals, and generate the medical image of the target from the unit signals classified into the groups.

## Description

### BACKGROUND

### 1. Field

The present disclosure relates to methods and apparatuses for generating an image in positron emission tomography.

### 2. Description of the Related Art

A medical image device is used to diagnose a patient by obtaining information about the patient via an image of functional processes in the human body. Methods of capturing a medical image have actively been developed and are currently used in hospitals. Such methods are largely divided into methods to obtain an anatomical image and methods to obtain a physiological image. Examples of a photographing technology that provides a detailed, high resolution anatomical image of the human body include magnetic resonance imaging (MRI) and computed tomography (CT). In this photographing technology, a 2-dimensional (2D) image of a cross-section of the human body or a 3-dimensional (3D) image of the human body or a part thereof using several 2D high-resolution images is generated to show accurate locations and shapes of various organs in the human body. An example of technology to obtain a physiological image includes positron emission tomography (PET). The PET can be used to diagnose a metabolic disorder by obtaining an image of the metabolic process in the human body.

PET is a photographing technology in which special radioactive tracers emitting positrons are generated as components during a metabolic process in the human body. The tracers are injected into the human body via an intravenous injection or inhalation. An external device is used to obtain locations of the tracers once injected into the human body. The external device detects two gamma rays of 511 eV emitted in opposite directions when the positrons emitted from the tracers and electrons combine with each other. The external device observes a distribution form and a change of a distribution aspect during a period of time.

Generally, a signal detector would process the gamma rays to later produce an image of the organ being targeted. However, signal dispersion or attenuation allows only a remarkably small amount of gamma rays, smaller than an actual amount of gamma rays emitted from the tracers injected into a target, to reach the signal detector. Accordingly, in order to obtain a sufficient amount of gamma rays to generate an image, a relatively long detection time in units of several minutes is required. However, because an organ of a patient moves in a relatively short period due to breath or heart beat, when the targeted organ is photographed in units of several minutes, the motion of such target affects the acquired image, thereby producing an image that is blurry and smudged. This phenomenon that affects the image due to a relative movement between a photographing apparatus and the target is referred to as motion blur, which is the main cause of reduced resolution of positron emission tomography.

### SUMMARY

Provided is a method and apparatus to generate an image in positron emission tomography, in which detected data is accurately classified to obtain a still image having high resolution.

Provided is a computer program embodied on a non-transitory computer-readable recording medium configured to control a processor to execute a method to generate an image in positron emission tomography.

Additional aspects will be set forth in part in the description which follows and, in part, will be apparent from the description, or may be learned by practice of the various embodiments.

In accordance with an illustrative configuration, there is provided a method to generate a medical image. The method includes dividing detected signals into sections at time intervals, wherein the detected signals are emitted from tracers introduced into a target. The method includes generating unit signals for each of the sections by accumulating the divided signals at each respective section. The method also includes classifying the unit signals into groups based on characteristics of each of the unit signals, and generating the medical image of the target from the unit signals classified into the groups.

The generating includes respectively generating 2-dimensional (2D) sinograms for each of the sections using each of the unit signals. The classifying includes classifying the 2D sinograms into the groups based on characteristics of the 2D sinograms. The sinograms for each of the sections may have similar, same, or different shapes according to a movement of the target including the tracers.

The characteristics are gradients indicating 2D gradients of the 2D sinograms.

The classifying includes calculating feature values indicating the characteristics of the unit signals and classifying the unit signals into the groups based on the calculated feature values.

The classifying further includes calculating the feature values from a correlation value indicating similarity between the unit signals.

The classifying further includes determining a maximum value and a minimum value of the feature values and respectively assigning a number of sections to the groups between the maximum value and the minimum value. The unit signals are classified into the respective number of sections assigned to the groups including the feature values of the unit signals.

The classifying further includes listing the unit signals based on results of comparing the feature values. The unit signals are classified into the plurality of groups based on a listed order.

The classifying is performed using a k-means clustering algorithm.

The generating includes generating the medical image of the target from the unit signals by registering the unit signals such that locations of the tracers, indicated by the groups, match.

Feature values of unit signals may be extracted and unit signals having similar feature values may be accumulated. As a result, an accurate registration of the unit signals is possible because the signal to noise ratio, SNR, increases as unit signals are accumulated. Also, when the unit signals are accumulated, time required to register the unit signals may be reduced.

The method is further configured to include estimating movement information of the tracers from a location of a tracer indicated by a reference group, from among the groups, to a location of a tracer indicated by each of the groups. The generating includes generating the medical image of the target from the unit signals by registering the unit signals based on the movement information. The movement information may reflect information about a location change of the tracer due to a movement of the target.

The method estimates the movement information based on a result of comparing the unit signals assigned to the reference group with the unit signals assigned to the each of the plurality of groups.

The movement information is estimated by analyzing the unit signals classified into groups without a help of an external apparatus, such as a motion sensor. Because unit signals are classified based on characteristics of the unit signals indicating locations of tracers without using an external device, adverse effects related to using an external device may be prevented. Thus, an accurate image registration is possible. As a result, a clear still image may be generated.

The method estimates the movement information based on a result of comparing a sinogram obtained by accumulating the unit signals assigned to the reference group and a sinogram obtained by accumulating the unit signals assigned to each of the groups.

The classifying includes classifying the unit signals into the groups based on threshold values, based on lower difference between feature values, which indicate the characteristics of the unit signals, of sinograms in one group, or based on a data clustering algorithm.

When the unit signals are classified into groups based on characteristics of the unit signals, regardless of phase information, unit signals indicating the same location may be classified in one group. Thus, the total number of groups is reduced. As a result, the number of operations for movement estimation and image generation may be reduced.

In accordance with another illustrative configuration, there is provided an apparatus to generate a medical image. The apparatus includes a unit signal generator configured to divide detected signals into sections at time intervals and generate unit signals for each of the sections by accumulating the divided signals at each respective section, wherein the detected signals are emitted from tracers introduced into a target. The apparatus further includes a classifier configured to classify the unit signals into groups based on characteristics of each of the unit signals. The apparatus further includes an image generator configured to generate the medical image of the target from the unit signals classified into the groups.

The unit signal generator is further configured to generate 2-dimensional (2D) sinograms for each of the sections using each of the unit signals. The classifier is further configured to classify the 2D sinograms into the groups based on characteristics of the 2D sinograms.

The characteristics are gradients indicating 2D gradients of the 2D sinograms.

The classifier calculates feature values indicating the characteristics of the unit signals and classifies the unit signals into the groups based on the calculated feature values.

The feature values are calculated from a correlation value indicating similarity between the unit signals.

The classifier is further configured to determine a maximum value and a minimum value of the feature values and respectively assigning a number of sections to the groups between the maximum value and the minimum value. The unit signals are classified into the respective number of sections assigned to the groups including the feature values of the unit signals.

The classifier is further configured to list the unit signals based on results of comparing the feature values. The unit signals are classified into the groups based on a listed order.

The classifier uses a k-means clustering algorithm.

The image generator generates the medical image of the target from the unit signals by registering the unit signals such that locations of the tracers, indicated by the groups, match.

The apparatus further includes a movement estimator configured to estimate movement information of the tracers from a location of a tracer indicated by a reference group, from among the groups, to a location of a tracer indicated by each of the groups. The image generator generates the medical image of the target from the unit signals by registering the unit signals based on the movement information.

The movement information is estimated based on a result of comparing the unit signals assigned to the reference group with the unit signals assigned to each of the plurality of groups.

The movement information is estimated based on a result of comparing a sinogram obtained by accumulating the unit signals assigned to the reference group and a sinogram obtained by accumulating the unit signals assigned to each of the groups.

The classifier is further configured to classify the unit signals into the groups based on threshold values, based on lower difference between feature values, which indicate the characteristics of the unit signals, of sinograms in one group, or based on a data clustering algorithm.

Detected data is classified without using an external device and the classified data is registered to generate a PET medical image through an image registration method. As a result, data can be accurately classified and a still image having high resolution can be generated. To generate the still image, the image generator may register unit signals of the groups such that a location of a tracer in each group matches a location of a tracer in a reference group. The image generator may thus generate the still image without motion blur from all the unit signals included in the groups.

In accordance with an illustrative configuration, there is further provided a signal detector configured to detect the signals emitted from the tracers injected into the target.

In accordance with another illustrative configuration, there is provided a computer program embodied on a non-transitory computer readable medium configured to control a processor to perform the method as described above.

### BRIEF DESCRIPTION OF THE DRAWINGS

These and/or other aspects will become apparent and more readily appreciated from the following description of the embodiments, taken in conjunction with the accompanying drawings of which:

FIG. 1 a diagram of an apparatus for generating an image, according to an illustrative example;

FIG. 2 is a diagram illustrating a computer of FIG. 1 and a flow of data, according to an illustrative example;

FIG. 3 is a diagram illustrating an example of line of response (LOR) data, according to an illustrative example;

FIG. 4 is a diagram illustrating an example of LOR data shown in a sinogram, according to an illustrative example;

FIG. 5 is a diagram illustrating an example of classifying unit signals into a plurality of groups, according to an illustrative example;

FIG. 6 is a diagram illustrating another example of classifying unit signals into a plurality of groups according to feature values of the unit signals, according to an illustrative example;

FIG. 7 is a diagram illustrating another example of classifying unit signals into a plurality of groups according to feature values of the unit signals, according to an illustrative example; and

FIG. 8 is a flowchart illustrating a method of compensating for motion blur of an image, according to an illustrative example.

### DETAILED DESCRIPTION

The following detailed description is provided to assist the reader in gaining a comprehensive understanding of the methods, apparatuses, and/or systems described herein. Accordingly, various changes, modifications, and equivalents of the systems, apparatuses and/or methods described herein will be suggested to those of ordinary skill in the art. Also, descriptions of well-known functions and constructions may be omitted for increased clarity and conciseness.

The terminology used herein is for the purpose of describing particular embodiments only and is not intended to be limiting the present invention. As used herein, the singular forms "a," "an" and "the" are intended to include the plural forms as well, unless the context clearly indicates otherwise. It will be further understood that the terms "have" and/or "having" or "include" and/or "including" when used in this specification, specify the presence of stated features, integers, steps, operations, elements, and/or components, but do not preclude the presence or addition of one or more other features, integers, operations, elements, components, and/or groups thereof.

Unless otherwise defined, all terms (including technical and scientific terms) used herein have the same meaning as commonly understood by one of ordinary skill in the art to which the present invention belongs. It will be further understood that terms, such as those defined in commonly used dictionaries, should be interpreted as having a meaning that is consistent with their meaning in the context of the relevant art and will not be interpreted in an idealized or overly formal sense unless expressly so defined herein.

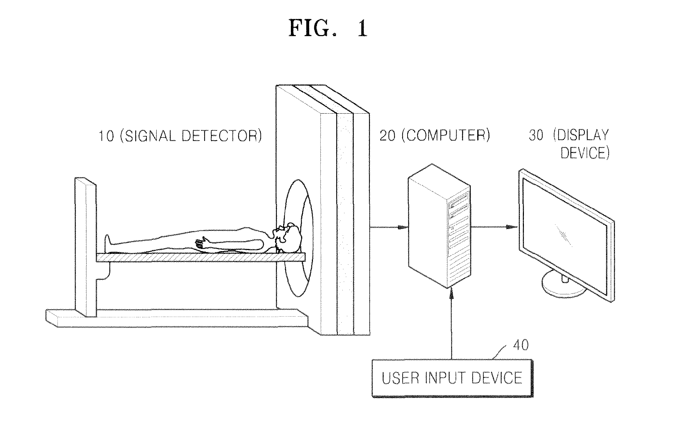

FIG. 1 a diagram of an apparatus for generating an image, according to an illustrative example. FIG. 1 illustrates an overall system to generate an image of a physical cross-section of a patient. Referring to FIG. 1, the apparatus includes, but it is not limited to, a signal detector 10, a computer 20, a display device 30, and a user input device 40.

The signal detector 10 detects a signal emitted from a tracer introduced into a target. The target may be a living organism, such as an animal or a person. When the target is a person, an operator injects the target with a special radioactive tracer via an intravenous injection. In the alternative, the target may inhale or swallow the tracer. The tracer emits a positron in a form of a component during a metabolic process.

The positron or anti-electron is an antiparticle or an antimatter counterpart of an electron. The positron is emitted from a radioactive isotope, such as C-11, N-13, O-15, or F-18. The special radioactive tracer may be generated by injecting the radioactive isotope as an element to be part of the patient's metabolism. An example of the special radioactive tracer that may be used includes a glucose-like material referred to as F-18-FDG. When the glucose-like material is injected in the human body, tracers concentrate in a region where glucose metabolism is concentrated, such as a cancerous area in the body.

Continuing with FIG. 1, the injected tracer emits a positron, and as the emitted positron is combined with an electron, two gamma rays of 511 eV are emitted in opposite directions. The signal detector 10 detects the gamma rays, and transmits data about the detected gamma rays to the computer 20 in a form of line of response (LOR).

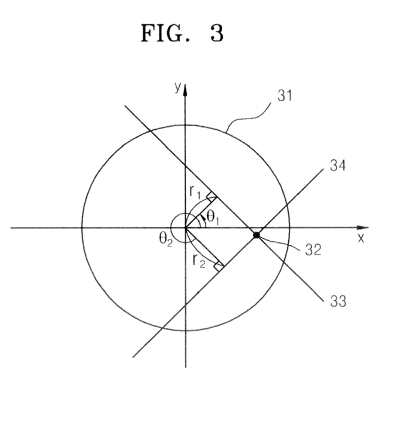

FIG. 3 is a diagram illustrating an example of LOR data, in accordance with an illustrative configuration. LOR data is a data form indicating a location of a straight line in a space. A tracer 32 is located in a detecting space of a scanner 31. Two gamma rays are emitted when positrons from the tracer 32 react with electrons. The two gamma rays are emitted in opposite directions along a straight line, that is, at 180° from each other. FIG. 3 illustrates two straight lines 33 and 34, i.e., two gamma rays as reactions of positrons and electrons. Referring to the straight line 33, when a perpendicular line is drawn on the straight line 33 starting from on an origin of the scanner 31, a distance from the origin of the scanner to the straight line 33 is defined as r₁ and an angle between the perpendicular line on the straight line 33 and an x-axis of the scanner 31 is defined as θ₁. As a result, the LOR data corresponding to the straight line 33 is defined as (r₁, θ₁). Similarly, referring to the straight line 34, when a perpendicular line is drawn on the straight line 34 starting from the origin in the scanner 31, a distance from the origin of the scanner 31 to the straight line 34 is defined as r₂ and an angle between the perpendicular line on the straight line 34 and the x-axis of the scanner 31 is defined as θ₂. As a result, the LOR data corresponding to the straight line 34 is defined as (r₂, θ₂). As described above, when two or more LOR data are obtained, a location of the tracer 32 may be determined to be at an intersection point of the two or more LOR data.

With respect to the illustrative example described in FIG. 3, the signal detector 10 of FIG. 1, transmits the LOR data, (r₁, θ₁) and (r₂, θ₂), of the detected gamma rays, straight line 33 and straight line 34, to the computer 10. Based on a location of the tracer 32 determined, detected, or defined from the LOR data, the computer 20 generates an image of the organ through the display device 30. The display device 30 displays the image generated from the computer 20 on a display panel.

Through the user input device 40, the operator may input information required to operate the computer 20, such as commands to start and stop the computer 20. In an alternative configuration, the operations to run the computer 20 may be obtained from a storage device, instead of the user input device 40.

In one illustrative example, the signal detector 10, the computer 20, the display device, and the user input device 40, each may be implemented using one or more general-purpose or special purpose computers, such as, for example, a processor, a controller and an arithmetic logic unit, a digital signal processor, a microcomputer, a field programmable array, a programmable logic unit, a microprocessor or any other device capable of responding to and executing instructions in a defined manner. The signal detector 10, the computer 20, the display device, and the user input device 40, each may run an operating system (OS) and one or more software applications that run on the OS. The processing device may also access, store, manipulate, process, and create data in response to execution of the software. For purpose of simplicity, in the description of the signal detector 10, the computer 20, the display device, and the user input device 40, each is used as singular; however, one skilled in the art will appreciated that a processing device may include multiple processing elements and multiple types of processing elements. For example, the signal detector 10, the computer 20, the display device, and the user input device 40, each may include multiple processors or a processor and a controller. In addition, different processing configurations are possible, such a parallel processors.

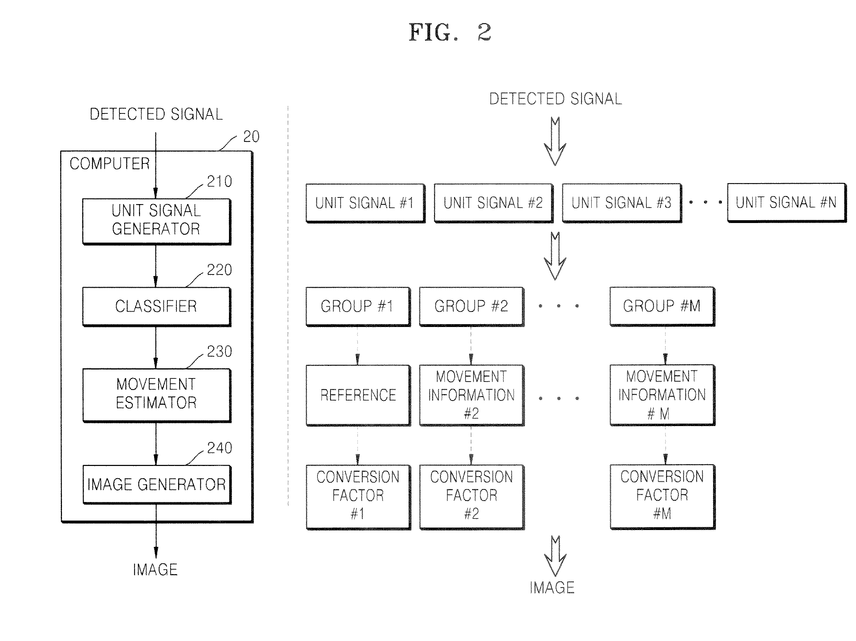

FIG. 2 is a diagram illustrating the computer 20 of FIG. 1 and a flow of data in accord with an operation of the computer 20. Referring to FIG. 2, the computer 20 includes, but it is not limited to, a unit signal generator 210, a classifier 220, a movement estimator 230, and an image generator 240. The left diagram in FIG. 2 shows a flow of data according to an operation of each of the unit signal generator 210, the classifier 220, the movement estimator 230, and the image generator 240.

The unit signal generator 210 obtains the signal detected by the signal detector 10 of FIG. 1, divides the obtained signal into sections at a predetermined time interval, and generates a unit signal for each section by accumulating divided signals from each section. For example, signals detected by the signal detector 10 may be divided into predetermined time intervals, after being processed in a time order of detection. Each unit signal may then be generated by accumulating the divided signals at each respective predetermined time interval. The generated unit signals may also be listed in a time order. Referring to the flow of data in FIG. 2, a total number of N unit signals, from a unit signal #1 to a unit signal #N, listed in a time order are shown at the right side of the unit signal generator 210. In one configuration, the predetermined time interval between each section may be configured to be of equal time duration or may be configured to be of different time durations. Thus, signals detected by the signal detector 10 may be divided into sections corresponding to predetermined time intervals, wherein the predetermined time intervals may have equal length in time or different lengths in time.

When the signal detected by the signal detector 10 is a LOR, a location of a tracer may not be determined using one LOR. In some instances, reliability of the location of the tracer may be low when the number of LORs is small. Accordingly, the unit signal generator 210 may determine sections at one or more predetermined time intervals so that a sufficient amount of data is accumulated to determine an intersection point of LOR data as the location of the tracer. However, when the predetermined time interval of the divided signals increases, it may be difficult to accurately determine the location of the tracer due to a movement of the target organ. Accordingly, an amount of time for each predetermined time interval may be determined considering a degree of the movement of the target, a movement period of the target, or a time interval for scanner to detect the LOR. In one illustrative example, the sections are short time intervals, for example, time intervals of less than or equal to 1 second, but the sections are not limited thereto. In one example, noise may occur in a unit signal generated by accumulating signals during the predetermined time interval. To reduce the effect of the noise, histogram clipping may be applied to the unit signal. The histogram clipping is a method used to determine a minimum value and a maximum value of a signal expressed in a histogram and defines limiting values of the signal to be between the minimum value and the maximum value. Accordingly, a value lower than the minimum value is limited to the minimum value and a value higher than the maximum value is limited to the maximum value.

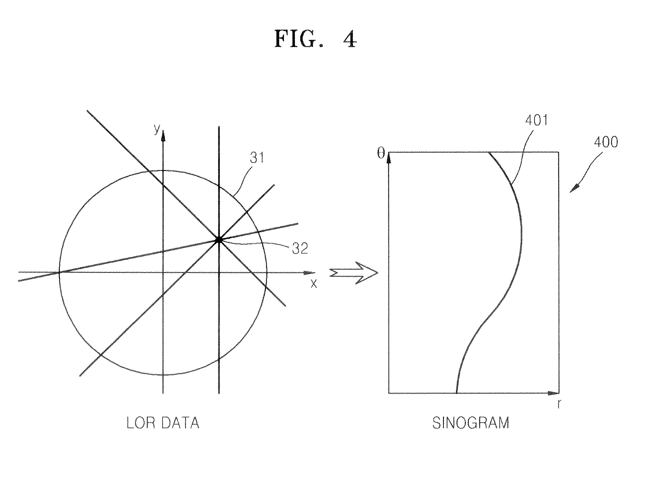

The unit signal generated as previously described may include several LORs, and a sinogram may be used to accumulate the LORs. A method of showing LOR data in a sinogram will now be described with reference to FIG. 4.

FIG. 4 is a diagram illustrating an example of LOR data shown in a sinogram, in accordance with an illustrative example. Projection information may be expressed in the sinogram, in which a horizontal axis is r and a vertical axis is θ. Because the LOR data described above also includes r and θ as variables, the LOR data may be illustrated in the sinogram. The LOR data having a value of (r, θ) corresponds to (r, θ) coordinates on the sinogram. A graph 40 is a sinogram of LORs of several gamma rays emitted from the tracer 32 in a detecting space of the scanner 31. The location of the tracer 32 in the detecting space of the scanner 31 corresponds to a curve 41 in the graph 40. Accordingly, when tracers are in different coordinates, a sinogram of signals detected from the tracers may include several curves.

Thus, the unit signal generator 210 of FIG. 2 may generate a sinogram by accumulating LORs included in each unit signal. In one example, the unit signal generator 210 may generate a sinogram according to unit signals. In this example, the sinograms may have similar, same, or different shapes according to the movement of the target including the tracer 32.

Referring back to FIG. 2, the classifier 220 classifies the unit signals into groups based on characteristics of the unit signals generated by the unit signal generator 210. For instance, referring to the flow of data of FIG. 2, a total number of M groups from group #1 to group #M are shown. M denotes a number smaller than N, which is a total number of unit signals. In one illustrative example, M may be defined to be 5 or above, but is not limited thereto. M may be determined based on a calculation performance of a computer or required resolution of a final image.

The location of the tracer indicated by each unit signal may differ according to a movement of the target. In order to generate one still image, all the unit signals would be added and registered such that the locations of the tracers indicated by the unit signals match. However, it may be difficult to register the unit signals due to a low signal to noise ratio (SNR) of the unit signals. To overcome this difficulty, a plurality of unit signals may be accumulated to improve SNR. For example, feature values of unit signals may be extracted and unit signals having similar feature values may be accumulated. As a result, an accurate registration of the unit signals is possible because the SNR increases as unit signals are accumulated. Registering a unit signal may comprise processing the unit signal, in particular to match the locations of the tracers indicated by the unit signals, i.e. performing a motion compensation processing, for the generation of the medical image.

Also, when the unit signals are accumulated, time required to register the unit signals may be reduced. It may take a long time to register all unit signals. For example, when N unit signals are registered, a total number of N-1 operations may need to be performed based on one unit signal. This may be due to matching operations of the location of the tracer indicated by the one unit signal with N-1 locations of the tracers indicated by the remaining N-1 unit signals.

On the other hand, unit signals at the same location may be gathered as one group, and groups may be registered to reduce the total time. For example, when M groups are registered, a total of M-1 operations may need to be performed based on one group. In one illustrative example, M denotes a number smaller than N, which is the total number of unit signals. Also, a time to register the groups may be shorter than the time to register the unit signals.

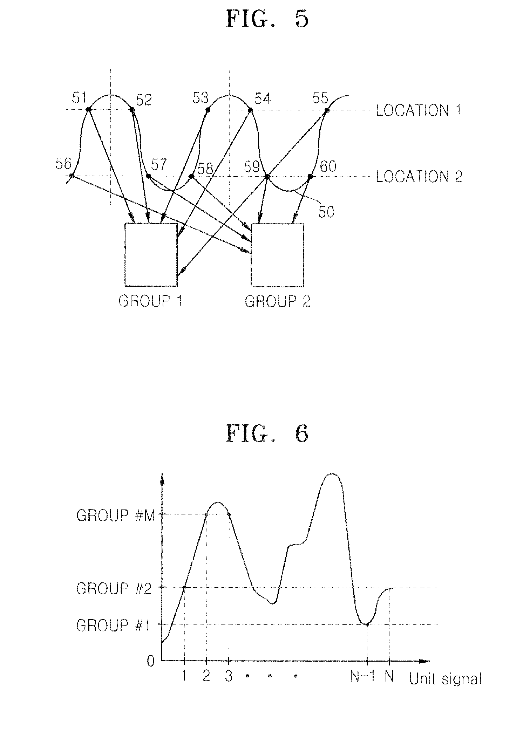

FIG. 5 is a diagram illustrating an example of the classifier 220 classifying unit signals into a plurality of groups, in accordance with an illustrative configuration.

A graph 50 of FIG. 5 shows each unit signal indicating a location change of a tracer when the unit signals are listed in a detected time order, i.e. in the time order of their detection. As shown in FIG. 5, in the detecting space of the scanner 31, the classifier 220 of FIG. 2 classifies unit signals 51 through 55 at a location 1 of the tracer to a group 1. Also, in the detecting space of the scanner 31, the classifier 220 classifies unit signals 56 through 60 at a location 2 of the tracer to a group 2. In this example, the tracer is alternately shown in the location 1 and the location 2 because the tracer injected into an organ may move along with a periodical movement of the organ. In one illustrative example, when unit signals are classified with respect to M locations, the classifier 220 may generate a total of M groups.

Based on results of analyzing the unit signals, the classifier 220 classifies the unit signals into groups by gathering unit signals at the same location and in one group. When a unit signal is shown in a sinogram, a location of a tracer indicated by the unit signal corresponds to a curve on the sinogram. As a result, the classifier 220 may classify unit signals according to the location of the tracer based on similarities between sinograms and characteristics of the sinograms of the unit signals.

According to such configuration performed by the apparatus of FIG. 1 to classify the unit signals, the total number of groups may be reduced. For example, locations of tracers may be the same even when the locations have different phases in a movement period of a target.

Hereinafter, 'phase information' is defined as information about a phase of a detecting time of data in a movement period of the target. Referring to FIG. 5, when the unit signals are classified using phase information, the unit signals 51, 53, and 55 may be classified in one group because they are in the same phase. The unit signals 52 and 54 may be classified into another group because they are both in the same phase.

However, when the classifier 220 classifies the unit signals based on characteristics of the unit signals, regardless of phase information, in a period as described above, the unit signals 51 through 55 at the same location may be classified in one group. The unit signals 51 through 55 would be classified in one group despite that the phases of the unit signals 51, 53, and 55 and the phases of the unit signals 52 and 54 are different from each other. Thus, the total number of groups generated by the classifier 220 is reduced. As a result, the number of operations that the movement estimator 230 and image generator 240 perform may be reduced.

The classifier 220 may calculate a feature value of a sinogram to determine similarity between sinograms based on characteristics of the sinograms of the unit signals. For example, the classifier 220 may calculate a feature value of a sinogram of each unit signal and classify the unit signals into the plurality of groups by gathering unit signals having similar feature values of sinograms as one group. Because a sinogram is represented in a 2-dimensional (2D) graph, various feature values capable of determining the similarity of 2D graphs may be used. In one example of a feature value, the classifier 220 may use a correlation of sinograms of unit signals. Accordingly, the classifier 220 may classify the unit signals into a plurality of groups by gathering unit signals having a low correlation value or high correlation as one group.

Alternatively, the classifier 220 may calculate as a feature value a result of applying a predetermined 2D filter to a sinogram of each unit signal and may classify unit signals into a plurality of groups by gathering unit signals having similar feature values as one group. In one example, a Gabor filter may be used as the predetermined 2D filter, but any other 2D filter may be used.

Alternatively, the classifier 220 may generate a gradient that is a 2D differential image indicating a 2D grade of a sinogram, calculate a feature value of the gradient, and classify unit signals into a plurality of groups by gathering unit signals having similar feature values of gradients in one group. The classifier 220 may perform other similar processes to calculate a feature value of an image.

Unit signals may be classified according to similar feature values by configuring the classifier 220 or a method to classify unit signals into a plurality of groups by gathering unit signals having predetermined threshold values or lower difference, i.e. differences smaller than a predetermined value, between the feature values of sinograms in one group, or by using one of various data clustering algorithms. For example, a k-means clustering algorithm may be used in which given data is quickly and effectively clustered into k groups. In one example, the classifier 220 may calculate a feature value of sinogram of each unit signal and classify unit signals into a plurality of groups by clustering the unit signals using the k-means clustering algorithm. Alternatively, any other classifying algorithm may be used, such as a Gaussian mixture model analysis method, a principal components analysis (PCA) method, or a linear discriminant classification (LDC) method.

Alternatively, the classifier 220 may use a gradient vector flow (GVF) snake algorithm to classify unit signals according to characteristics of sinograms of the unit signals. A snake algorithm is an algorithm to detect an edge in an image. The GVF snake algorithm is a type of snake algorithm that detects an edge in an image from a distribution of gradient vectors of the edge. The GVF snake algorithm may be used to extract characteristics of the image from the detected edge. When such a GVF snake algorithm is applied to a sinogram that is a 2-dimensional (2D) graph, the classifier 220 classifies unit signals based on characteristics of sinograms of the unit signals generated using the GVF snake algorithm. The GVF snake algorithm shows a satisfactory performance even in a sinogram having a low SNR, and is suitably applied to a sinogram having a shape of a sine function.

FIG. 6 is a diagram illustrating another example of classifying unit signals into a plurality of groups according to feature values of the unit signals, in accordance with an illustrative example. Referring to FIG. 6, the classifier 220 may obtain a minimum value and a maximum value of the feature values, obtain M sections between the minimum value and the maximum value, and classify each unit signal N into a total of M groups by gathering unit signals having feature values corresponding to each section as one group.

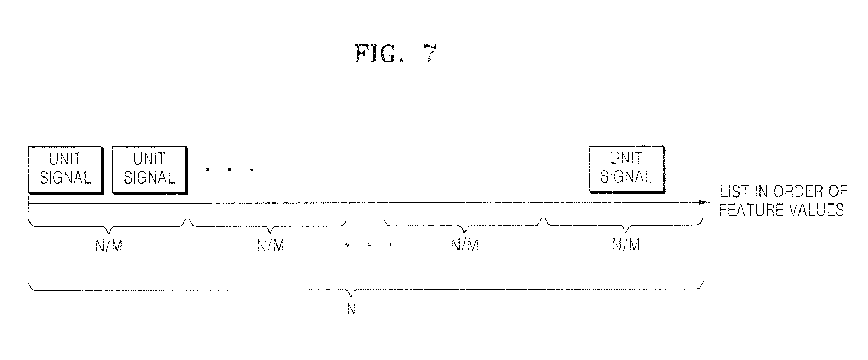

FIG. 7 is a diagram illustrating another example of classifying a unit signal into a plurality of groups according to feature values of the unit signal, in accordance with an illustrative example. Referring to FIG. 7, the classifier 220 lists the unit signals in an order of the feature values and classifies the unit signals according to a listed order into a total of M groups by gathering N/M unit signals in one group. Thus, the classifier 220 may divide the ordered list into sections of N/M unit signals forming M groups.

Referring back to FIG. 2, the movement estimator 230 estimates movement information of a target according to the groups which the classifier 220 generated. For example, the movement estimator 230 estimates movement information of a target from a location of a tracer, indicated by a reference group from among the groups the classifier 220 generated, to a location of the tracer indicated by each group. Such movement information may reflect information about a location change of the tracer followed by a movement of the target. For example, when locations of tracers indicated by the groups are different due to the movement of the target, movement information of each group denotes a distance and direction of a location of a tracer that moved based on a location of the tracer indicated by a group #1. The movement information is estimated by analyzing the unit signals classified into groups without a help of an external apparatus, such as a motion sensor. Accordingly, the movement estimator 230 estimates the movement information based on a result indicative of a comparison between the unit signals assigned to one reference group and the unit signals assigned to each group.

The location of the tracer indicated by each group is determined from the sinogram. Accordingly, the movement estimator 230 estimates a location change of the tracer based on a result of comparing a sinogram, which is obtained by accumulating the unit signals assigned to one reference group, with another sinogram, which is obtained by accumulating the unit signals assigned to each group. As a result, the movement estimator 230 estimates the movement information from the estimated location change.

The movement estimator 230 determines a group #1 from among M groups as a reference group and estimates movement information of each group from the group #1. As such, an optical flow may be used to estimate movement information of the target in an image. The optical flow would estimate a motion vector distribution of an object or sensor on coordinates or a snake algorithm to detect an edge or outline of an image, which is similar to the optical flow. Alternatively, the movement estimator 230 may use any one of various methods to estimate movement information in an image.

Referring to the right drawing of FIG. 2, movement information #2 denotes a location change of a tracer from a location of a tracer indicated by a group #1, which is a reference group, to a location of a tracer indicated by a group #2. Also, although not illustrated, movement information #3 corresponding to a group #3 denotes a location change of a tracer from the location of the tracer indicated by the group #1 to a location of a tracer indicated by the group #3. Similarly, movement information #M denotes a location change of a tracer from the location of the tracer indicated by the group #1 to a location of a tracer indicated by a group #M.

An example of a method of estimating movement information of a tracer in a 3D space will now be described.

When the detecting space in the scanner 31 is 3D, the location of the tracer may be represented in a 3D space by obtaining a signal on a 2D plane (x-y plane) with respect to different z values on a z-axis, which is an axis direction of the scanner 31 if the scanner 31 is cylindrical. Thus, the signal detector 10 may repeatedly obtain the signal on the 2D plane (x-y plane) with respect to the different z values while moving in a z-axis direction, or may simultaneously obtain the signal on the 2D plane (x-y plane) with respect to the different z values.

In one illustrative example, the unit signal generator 210 generates N unit signals by accumulating the signals obtained as described above, and the classifier 220 classifies the N unit signals into M groups by gathering unit signals having the same or similar location of the tracer in the 3D space into one group. For each group, the movement estimator 230 estimates movement information of the tracer in 3D according to the groups. For example, the movement estimator 230 may estimate the movement information of the tracer in 3D from a 3D location of the tracer indicated by a reference group, among the groups generated by the classifier 220, to a 3D location of the tracer indicated by each of the remaining groups.

For example, group #1 is a reference group and movement information #2 indicates a location change from a 3D location of the tracer indicated by the group #1 to a 3D location of the tracer indicated by a group #2. If the movement information #2 is estimated, the movement estimator 230 may first estimate a movement in a z-axis direction from movements of the tracer in 3D, and then estimate a movement in an x-y plane (or a z plane) direction. The movement information of each group may be expressed as a 3D vector.

In addition, when the location of the tracer is identical in a plane where a value of a z-axis is a constant k in the reference group #1 and in a plane where a value of a z-axis is a constant k+a (a>0) in the group #2, while estimating the movement information #2, the movement estimator 230 may determine that the tracer moved in a positive direction on the z-axis. As such, the movement estimator 230 may determine a direction of a movement vector in the z-axis direction of the movement information #2 and determine a size of the movement vector in the z-axis direction from a size of constant a, thereby estimating the movement information #2 in the z-axis direction. Other similar examples to estimate the movement information #2 in the z-axis direction may be implemented.

Once the movement information #2 in the z-axis direction is estimated, the movement estimator 230 may estimate the movement information #2 on the z plane. Accordingly, the movement estimator 230 estimates the movement information of the tracer on any representative z plane to estimate movement information of the tracer in an x-y direction. Alternatively, the movement estimator 230 may estimate the movement information of the tracer on a plurality of z planes to estimate movement information of the tracer in the x-y direction.

An example of the movement estimator 230 estimating the movement information of the tracer in the x-y direction, with respect to each of the plurality of z planes on the detecting space of the scanner 31, will now be described. In one example, in a z plane where z=1 is a first plane and the movement information #2 is to be estimated, the movement estimator 230 estimates movement information of the tracer on the first plane from the group #1 to the group #2 by estimating a location change of the tracer. The movement estimator 230 estimates the location change from a location of the tracer indicated by the first plane of the reference group #1 to a location of the tracer indicated by the first plane of the group #2.

Similarly, the movement estimator 230 estimates the movement information of the tracer on a second plane where z=2. By repeatedly performing such a method on each z plane, the movement estimator 230 estimates the movement information of the tracer on each of the plurality of z planes. Accordingly, on the plurality of z planes, the movement estimator 230 estimates the movement information of the movement information #2 in the x-y direction from the movement information of the tracer.

The movement estimator 230 generates the movement information #2 of the tracer by combining the movement information of the tracer in the x-y direction and the movement information of the tracer in the z-axis direction, estimated as described above. By performing such a method on each group, the movement estimator 230 generates movement information #2 to movement information #M for the M groups.

In one illustrative example, the movement estimator 230 estimates the movement information on the 2D plane using an optical flow to estimate a motion vector distribution of an object or a sensor in coordinates as described above, or a snake algorithm to detect an edge of an image. However, any one of other various methods may be applied to estimate movement information (motion estimation) in an image.

The movement estimator 230 may use a histogram distribution of a sinogram of each plane to estimate the movement information on the 2D plane. For example, the movement estimator 230 represents each of a 2D sinogram of the first plane of the group #1 and a 2D sinogram of the first plane of the group #2 in a histogram distribution to estimate the movement information #2 on the x-y plane. A histogram distribution is an example of a method to indicate, identify, or illustrate characteristics of a sinogram. As a result, the histogram distribution may be used for the movement estimator 230 to estimate the movement information in the z-axis direction. Also, the classifier 220 may use the histogram distributions of the sinograms of the unit signals to gather the unit signals having similar histogram distributions. A method of expressing a 2D image or a 2D graph in a histogram distribution would be apparent in light of the descriptions provided above in reference to the movement estimator 230.

As described above, the movement estimator 230 may estimate the movement information of each group in the z-axis direction through the histogram distribution of the sinogram, and estimate the movement information on the x-y plane.

The image generator 240 generates a medical image on the target from the unit signals classified into the groups by the classifier 220. For example, the image generator 240 generates a medical image on the target from the unit signals by registering the unit signals included in the groups based on the movement information of each group estimated by the movement estimator 230.

For example, the image generator 240 generates a still image from each group based on the movement information estimated by the movement estimator 230 and based on each of the M groups generated by the classifier 220. Accordingly, the image generator 240 generates a conversion factor including the movement information of a group according to the groups. The conversion factor may be used as a variable while generating an image. The conversion factors of the groups are illustrated in FIG. 2.

The image generator 240 generates an image by repeatedly performing iterative reconstruction by reflecting movement information. For example, the image generator 240 generates a still image by converting all unit signals using the conversion factors including movement information of the groups as variables of an image generating algorithm. Also, to generate the still image, the image generator 240 registers unit signals of the groups such that a location of a tracer in each group matches a location of a tracer in a group #1, which is a reference group.

The iterative reconstruction is an example of an algorithm to estimate an input signal when a transfer function and an output signal are known. In other words, the iterative reconstruction is repeatedly performed as the input signal changes until the output signal becomes a desired output signal. The iterative reconstruction includes setting an initial value of the input signal to have a predetermined value and then applying a transfer function to the input signal.

To further illustrate the iterative reconstruction, in positron emission tomography (PET), an LOR signal obtained from the signal detector 10 is an input signal and an image generated from the input signal is an output signal. Accordingly, a system matrix to reconstruct an image from an LOR signal may be a conversion factor, which is a transfer function. In order to reconstruct one still image simultaneously from a plurality of group signals indicating locations of different tracers, the image generator 240 includes a conversion factor according to groups. In one configuration, to generate the still image, the image generator 240 reflects the movement information of each group to the conversion factor of each group to register the locations of the tracers of the groups while reconstructing the image. For example, when the group #1 is set as a reference group by the movement estimator 230, the image generator 240 uses a conversion factor #2 as a variable of an image generating algorithm while unit signals classified as the group #2 are reconstructed into an image. Similarly, the image generator 240 may use a conversion factor #M as a variable of an image generating algorithm while reconstructing unit signals classified as the group #M into an image. Because each conversion factor includes movement information of each group, the image generator 240 may generate a still image without motion blur from all the unit signals included in M groups.

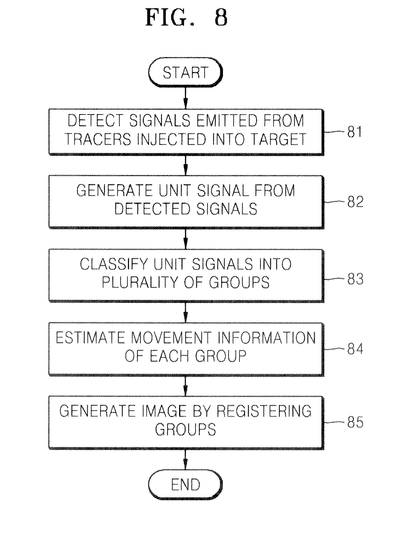

FIG. 8 is a flowchart illustrating a method of compensating for motion blur of an image, according to an illustrative configuration. The apparatus of FIGS. 1 and 2 may be implemented to perform the method to compensate for an image as described with respect to FIG. 8. As shown in FIG. 8, at operation 81, the method is configured to detect signals emitted from tracers injected into a target. In other words, the method is configured to enable signal detector 10 to detect gamma rays emitted from the tracers injected into the target and transmit the gamma rays as LOR data to the unit signal generator 210 of the computer 20. At operation 82, the method is configured to generate a unit signal from the detected signals. In reference to FIG. 2, the method is configured to enable the unit signal generator 210 to obtain detected signals, divide the obtained signals into sections in predetermined intervals, and generate unit signals by accumulating signals in each of the sections. At operation 83, the method is configured to classify the unit signals into a plurality of groups. Also, in reference to FIG. 2, the method is configured to enable the classifier 220 to classify the unit signals into a plurality of groups based on characteristics of the unit signals generated by the unit signal generator 210.

At operation 84, the method is configured to estimate movement information of each group. The method is configured to enable the movement estimator 230 of FIG. 2 to estimate movement information of the tracers for each group generated by the classifier 220, without any help from an external apparatus, such as a motion sensor. At operation 85, the method is configured to generate an image by registering the groups. The method is configured to enable the image generator 240 to generate a medical image of the target by registering the groups based on the movement information of each group estimated by the movement estimator 230.

According to the above embodiments, while generating an image of a moving target via PET, a still image having higher resolution may be generated by accurately classifying unit signals based on characteristics of the unit signals. According to an illustrative example, unit signals are classified through phase information determined by matching the unit signals to breathing or heart being periods using an external device. The phase information is easily determined by synchronizing time information when the unit signals are detected and movement periods. However, an error may be generated because the breathing or heart beating periods do not accurately match the movement of a target or movement periods of tracers according to the movement of the target.

To resolve this error, according to illustrative examples described above with reference to FIGS. 1 to 8, because unit signals are classified based on characteristics of the unit signals indicating locations of tracers without using an external device, adverse effects related to using an external device may be prevented. Thus, an accurate image registration is possible. As a result, a clear still image may be generated.

Also, while classifying the unit signals, a user may pre-set a number of groups or input other adjusting factors to the user input device. As a result, an image having a quality desired by the user may be produced according to a trade-off relationship between the quality of image and an operation load of a computer.

As described above, according to the one or more illustrative examples, detected data is classified without using an external device and the classified data is registered to generate a PET medical image through an image registration method. As a result, data can be accurately classified and a still image having high resolution can be generated.

It is to be understood that in accordance with illustrative examples, the operations in FIG. 8 are performed in the sequence and manner as shown although the order of some steps and the like may be changed. In accordance with an illustrative example, a computer program embodied on a non-transitory computer-readable medium may also be provided, encoding instructions to perform at least the method described in FIG. 8.

Program instructions to perform the method of FIG. 8 described herein, or one or more operations thereof, may be recorded, stored, or fixed in one or more non-transitory computer-readable storage media. The program instructions may be implemented by a computer. For example, the computer may cause a processor to execute the program instructions. The media may include, alone or in combination with the program instructions, data files, data structures, and the like. Examples of non-transitory computer-readable media include magnetic media, such as hard disks, floppy disks, and magnetic tape; optical media such as CD ROM disks and DVDs; magneto-optical media, such as optical disks; and hardware devices that are specially configured to store and perform program instructions, such as read-only memory (ROM), random access memory (RAM), flash memory, and the like. Examples of program instructions include machine code, such as produced by a compiler, and files containing higher level code that may be executed by the computer using an interpreter. The program instructions, that is, software, may be distributed over network coupled computer systems so that the software is stored and executed in a distributed fashion. For example, the software and data may be stored by one or more computer readable recording mediums. Also, functional programs, codes, and code segments for accomplishing the example embodiments disclosed herein can be easily construed by programmers skilled in the art to which the embodiments pertain based on and using the flow diagrams and block diagrams of the figures and their corresponding descriptions as provided herein.

A number of examples have been described above. Nevertheless, it will be understood that various modifications may be made. For example, suitable results may be achieved if the described techniques are performed in a different order and/or if components in a described system, architecture, device, or circuit are combined in a different manner and/or replaced or supplemented by other components or their equivalents. Accordingly, other implementations are within the scope of the following claims.

## Claims

1. A method to generate a medical image, the method comprising:
dividing detected signals into sections at time intervals, wherein the detected signals are emitted from tracers introduced into a target;
generating unit signals for each of the sections by accumulating the divided signals at each respective section;
classifying the unit signals into groups based on characteristics of each of the unit signals; and
generating the medical image of the target from the unit signals classified into the groups.

2. The method as recited in claim 1, further comprising:
respectively generating 2-dimensional (2D) sinograms for each of the sections using each of the unit signals, and
wherein the classifying comprises classifying the 2D sinograms into the groups based on characteristics of the 2D sinograms.

3. The method as recited in claim 2, further comprising:
configuring the characteristics are gradients to indicate 2D gradients of the 2D sinograms.

4. The method as recited in any one of claims 1 to 3, wherein the classifying comprises
calculating feature values indicating the characteristics of the unit signals and classifying the unit signals into the groups based on the calculated feature values.

5. The method as recited in claim 4, wherein the classifying further comprises calculating the feature values from a correlation value indicating similarity between the unit signals.

6. The method as recited in claim 4, wherein the classifying further comprises
determining a maximum value and a minimum value of the feature values and respectively assigning a number of sections to the groups between the maximum value and the minimum value,
wherein the classifying classifies the unit signals into the respective number of sections assigned to the groups comprising the feature values of the unit signals, or
listing the unit signals based on results of comparing the feature values,
wherein the unit signals are classified into the plurality of groups based on a listed order.

7. The method as recited in any one of claim 1 to 6, wherein the classifying is performed using a k-means clustering algorithm.

8. The method as recited in any one of claim 1 to 7, wherein the generating comprises
generating the medical image of the target from the unit signals by registering the unit signals such that locations of the tracers, indicated by the groups, match.

9. The method as recited in claim 8, further comprising:
estimating movement information of the tracers from a location of a tracer indicated by a reference group, from among the groups, to a location of a tracer indicated by each of the groups,
wherein the generating comprises generating the medical image of the target from the unit signals by registering the unit signals based on the movement information.

10. The method as recited in claim 9, wherein the movement information is estimated based on a result of comparing the unit signals assigned to the reference group with the unit signals assigned to the each of the plurality of groups, or based on a result of comparing a sinogram obtained by accumulating the unit signals assigned to the reference group and a sinogram obtained by accumulating the unit signals assigned to each of the groups.

11. An apparatus to generate a medical image, the apparatus comprising:
a unit signal generator configured to divide detected signals into sections at time intervals and generate unit signals for each of the sections by accumulating the divided signals at each respective section, wherein the detected signals are emitted from tracers introduced into a target;
a classifier configured to classify the unit signals into groups based on characteristics of each of the unit signals; and
an image generator configured to generate the medical image of the target from the unit signals classified into the groups.

12. The apparatus as recited in claim 11, wherein the unit signal generator is further configured to generate 2-dimensional (2D) sinograms for each of the sections using each of the unit signals, and
wherein the classifier is further configured to classify the 2D sinograms into the groups based on characteristics of the 2D sinograms.

13. The apparatus as recited in claim 11 or 12, wherein the classifier calculates feature values indicating the characteristics of the unit signals and classifies the unit signals into the groups based on the calculated feature values.

14. The apparatus as recited in any one of claims 11 to 13, wherein the classifier is further configured to determine a maximum value and a minimum value of the feature values and respectively assigning a number of sections to the groups between the maximum value and the minimum value, and
wherein the unit signals are classified into the respective number of sections assigned to the groups comprising the feature values of the unit signals, or
to list the unit signals based on results of comparing the feature values, and wherein the unit signals are classified into the groups based on a listed order.

15. A computer program embodied on a non-transitory computer readable medium configured to control a processor to perform the method of any one of claims 1 to 10.
